# EUROPEAN PATENT APPLICATION

(11) **EP 3 685 846 A1**
(43) Date of publication of application: **29.07.2020**
(21) Application number: 19305097.8
(22) Date of filing: 24.01.2019
(51) Int. Cl.: A61K 38/12, A61K 31/4709, A61P 21/00, A61P 17/06

(54) **USE OF THIOSTREPTON OR ITS DERIVATIVES FOR THE TREATMENT OF GENETIC DISEASES LINKED TO A PROTEIN CONFORMATIONAL DISORDER**

(71) Applicant: Genethon, 91000 Evry (FR); Centre d'Etude des Cellules Souches (CECS), 91100 Corbeil Essonnes (FR); Institut National de la Santé et de la Recherche Médicale, 75013 Paris (FR); Universite d'Evry Val d'Essonne, 91025 Evry (FR)
(72) Inventor: RICHARD, Isabelle, 91100 CORBEIL ESSONNES (FR); NISSAN, Xavier, 94220 CHARENTON-LE-PONT (FR); HENRIQUES, Sara, 91000 EVRY (FR); HOCH, Lucile, 78180 MONTIGNY LE BRETONNEUX (FR)
(74) Representative: Cabinet Laurent & Charras

(57) **Abstract**

The present invention relates to the use of a cyclic thiopeptide, advantageously thiostrepton or its derivatives, for treating a genetic disease linked to a conformational disorder of at least one protein, said disorder causing the cellular degradation of the protein.

## Description

### TECHNICAL FIELD

The present invention provides new pharmacological tools for treating genetic diseases linked to a conformational disorder of at least one protein, said disorder causing the cellular degradation of the protein through the proteasome pathway.

### STATE OF THE ART

Limb Girdle Muscular Dystrophies (LGMD) are a heterogeneous group of muscular dystrophies sharing common clinical presentation of weakness affecting the shoulder and pelvic girdles. Within this group, sarcoglycanopathies are a subgroup caused by mutations in genes encoding the transmembrane protein sarcoglycan (SG) complex, located in the sarcolemma of striated muscle. Because the SG complex plays a key role in the maintenance of sarcolemma integrity during muscle contraction, mutations in these genes lead to muscular dysfunction. Four SGs have been described, namely, α-, β-, γ-, δ-SG, which cause different subtypes of LGMD: LGMD2D, LGMD2E, LGMD2C, LGMD2F, respectively. While the clinical features of these LGMDs are well described, the molecular mechanisms associated with sarcoglycanopathies remain poorly understood. Analyses of large cohorts of patients have revealed that hundreds of different missense or null mutations of these genes can lead to sarcoglycanopathies, with the most frequent type being the arginine-to-cysteine substitution at the 77th amino acid (R77C) in α-SG. This mutation leads to the production of a misfolded but still functional protein, recognized by the endoplasmic reticulum quality control (ERQC) system and degraded by the endoplasmic-reticulum-associated protein degradation (ERAD) pathway through the ubiquitin-proteasome system (Bartoli, M. et al., Human molecular genetics 17, 1214-1221 (2008), Gastaldello, S. et al., The American journal of pathology 173, 170-181 (2008)).

Although no treatment is currently available for LGMD2D, recent studies suggest that inhibition of the different steps in the endoplasmic reticulum (ER) degradation system could be effective in restoring mutated α-SG expression in the plasma membrane (Bartoli, M. *et al.* (2008), Gastaldello, S. *et al.* (2008)). Evidence in support of this strategy has been described by different groups, as revealed by the positive impact of kifunensine when targeting mannosidase I activity (Bartoli, M. *et al.* (2008)) or the use of the proteasome inhibitor MG132 (Gastaldello, S. *et al.* (2008)). Overall, these studies provide strong evidence that targeting cellular quality control or degradation pathways are of interest to rescue missense mutations that cause LGMD2D.

However, there is still a need to find new therapeutical approaches for treating said kind of genetic diseases.

### SUMMARY OF THE INVENTION

The inventors have shown that thiostrepton, reported as specifically inhibiting the 19S proteasome, is a potent candidate for treating genetic diseases linked to a conformational disorder of at least one protein, said disorder causing the cellular degradation of the protein: The present application reveals that it is efficient for a large spectrum of mutations while displaying low toxicity. Moreover, it has a complementary action with respect to classical proteasome inhibitors such as bortezomib so that their combination has a synergic effect.

### Definitions

The definitions below represent the meaning generally used in the context of the invention and should be taken into account unless another definition is explicitly stated.

In the frame of the invention, the articles "a" and "an" are used to refer to one or several (*i.e.,* at least one) of the grammatical object of the article. By way of example, "an element" means at least one element, i.e. one or more than one elements.

The terms "around", "about" or "approximately" as used therein when referring to a measurable value such as an amount, a temporal duration and the like should be understood as encompassing variations of ± 20% or ± 10%, preferably ± 5%, more preferably ± 1%, and still more preferably ± 0.1% from the specified value.

Intervals/ranges: throughout this disclosure, various aspects of the invention can be presented in the form of a value interval (range format). It should be understood that the description of values in the form of an interval is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

"Isolated" means altered or removed from its natural environment or state. For example, an isolated nucleic acid or peptide is a nucleic acid or peptide which has been extracted from the natural environment in which it is usually found whether this be in a plant or living animal for example. A nucleic acid or peptide for example which is naturally present in a living animal is not an isolated nucleic acid or peptide in the sense of the invention whereas the same nucleic acid or peptide partially or completely separated from other components present in its natural environment is itself "isolated" in the sense of the invention. An isolated nucleic acid or protein can exist in substantially purified form, or can exist in a non-native environment such as, for example, a host cell.

In the context of the present invention, the following abbreviations for the commonly occurring nucleic acid bases are used: "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The nucleotide sequence which codes for a protein or an RNA or cDNA may possibly contain introns.

The terms "encoding" or "coding for" refer to the inherent property of specific sequences of nucleotides in a polynucleotide, such as a gene, a cDNA or an mRNA, used as a matrix to produce other polymers and macromolecules in biological processes, having either a defined nucleotide sequence (for example rRNA, tRNA and mRNA), or a defined amino acid sequence and the biological properties resulting therefrom. A gene therefore codes for a protein if transcription and translation of the mRNA corresponding to this gene produces the protein in a cell or in another biological system. Both the coding strand, the nucleotide sequence of which is identical to the mRNA sequence and is usually provided in the sequence listings and databases, and the non-coding strand, used as the template for transcription of a gene or cDNA, may be referred to as coding for the protein or for other product of this gene or cDNA.

The term "polynucleotide" as used in the context of the invention is defined as a chain of nucleotides. In addition, nucleic acids are polymers of nucleotides. Thus, the terms nucleic acids and polynucleotides as used therein are interchangeable. It is well known in the field of molecular biology and genetic engineering that nucleic acids are polynucleotides which can be hydrolysed into the monomeric "nucleotides". Nucleotides can be hydrolysed into "nucleosides". As used in the context of the invention, polynucleotides include, but are not limited to all types of nucleic acid sequences, i.e. molecules of nucleic acids which can be obtained by any means available in the art, including recombinant methods, i.e. cloning of nucleic acid sequences from a recombinant library or a cell genome, using ordinary cloning technologies such as PCR or by synthesis.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein or peptide sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homomers, heteromers, variants of polypeptides, modified polypeptides, derivatives, analogs, fusion proteins, among others. The polypeptides include natural peptides, recombinant peptides, synthetic peptides, or a combination thereof.

A "variant", as used herein, refers to an amino acid sequence that is altered by one or more amino acids. The variant may have "conservative" changes, wherein a substituted amino acid has similar structural or chemical properties, e. g. replacement of leucine with isoleucine. A variant may also have "non-conservative" changes, e. g. replacement of a glycine with a tryptophan. Analogous minor variations may also include amino acid deletions or insertions, or both. Guidance in determining which amino acid residues may be substituted, inserted, or deleted without abolishing biological or immunological activity may be found using computer programs well known in the art.

The terms "identical" or "homologous" refer to the sequence similarity or sequence identity between two polypeptides or between two nucleic acid molecules. When a position in both of the two compared sequences is occupied by the same base or amino acid monomer subunit (for example when a position in each of the two DNA molecules is occupied by adenine), the molecules are then homologous or identical at that position. The percent of homology/identity between two sequences depends on the number of matching positions shared by the two sequences and represents this number divided by the number of positions compared, multiplied by 100. For example, if 6 of the 10 positions in two related sequences are identical, the two sequences are 60% identical. As a general rule, the comparison is made by aligning the two sequences in order to obtain maximum homology/identity.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

"Expression vector" refers to a vector comprising a recombinant polynucleotide comprising expression control sequences operatively linked to a nucleotide sequence to be expressed. An expression vector comprises in particular sufficient cis-acting elements for expression; other elements for expression can be supplied by the host cell or in an *in vitro* expression system. Expression vectors include all those known in the art, such as cosmids, plasmids (*e.g.,* naked or contained in liposomes) and viruses (*e.g.,* lentiviruses, retroviruses, adenoviruses, and adeno-associated viruses) that incorporate the recombinant polynucleotide.

The term "promotor" as used here is defined as a DNA sequence recognised by the cell synthesis machinery or introduced synthetic machinery, which is required to initiate specific transcription of a polynucleotide sequence.

As used herein, the term "promoter/regulatory sequence" means a nucleic acid sequence, which is required for expression of a gene product operably linked to the promoter/regulatory sequence. In some instances, this sequence may be the core promoter sequence and in other instances, this sequence may also include an enhancer sequence and other regulatory elements, which are required for expression of the gene product. The promoter/regulatory sequence may, for example, be one which expresses the gene product in a tissue specific manner.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics, which are normal or expected for one cell or tissue type, might be abnormal for a different cell or tissue type.

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is an animal, preferably a mammal, more preferably a human. It may also be a mouse, a rat, a pig, dog or non-human primate (NHP), such as the macaque monkey.

In the sense of the invention, a "disease" or "pathology" is a state of health of an animal in which its homeostasis is adversely affected and which, if the disease is not treated, continues to deteriorate. Conversely, in the sense of the invention, a "disorder" is a state of health in which the animal is able to maintain homeostasis but in which the state of health of the animal is less favourable than it would be in the absence of the disorder. Left untreated, a disorder does not necessarily result in deterioration in the state of health of the animal over time.

A disease or disorder is "alleviated" ("reduced") or "ameliorated" ("improved") if the severity of a symptom of the disease or disorder, the frequency with which such a symptom is experienced by the subject, or both of these, is reduced. This also includes the disappearance of progression of the disease, i.e. halting progression of the disease or disorder. A disease or disorder is "cured" ("recovered") if the severity of a symptom of the disease or disorder, the frequency with which such a symptom is experienced by the patient, or both, is eliminated.

In the context of the invention, a "therapeutic" treatment is a treatment administered to a subject who displays the symptoms (signs) of pathology, with the purpose of reducing or removing these symptoms. As used herein, the "treatment of a disease or disorder" means reducing the frequency or severity of at least one sign or symptom of a disease or disorder experienced by the subject. A treatment is said to be prophylactic when it is administered to prevent the development, spread or worsening of a disease, particularly if the subject does not have or does not yet have the symptoms of the disease and/or for which the disease has not been diagnosed.

As used herein, "treating a disease or disorder" means reducing the frequency or severity of at least one sign or symptom of a disease or disorder experienced by a subject. Disease and disorder are used interchangeably herein in the context of treatment.

In the sense of the invention, an "effective quantity" or an "effective amount" of a compound is that amount of compound which is sufficient to provide a beneficial effect to the subject to which the compound is administered. The expression "therapeutically effective quantity" or "therapeutically effective amount" refers to a quantity which is sufficient or effective to prevent or treat (in other words delay or prevent the development, prevent the progression, inhibit, decrease or reverse) a disease or a disorder, including alleviating symptoms of this disease or disorder.

### DETAILED DESCRIPTION OF THE INVENTION

The present invention relates to the use of a cyclic thiopeptide, advantageously thiostrepton or its derivatives, for treating a genetic disease linked to a conformational disorder of at least one protein, said disorder causing the cellular degradation of the protein.

More specifically and according to a first aspect, the present invention thus relates to a pharmaceutical composition comprising at least a cyclic thiopeptide, advantageously thiostrepton or its derivatives, for use in the treatment of genetic diseases linked to a conformational disorder of at least one protein, said disorder causing the cellular degradation of the protein.

In other words, a composition comprising a cyclic thiopeptide, advantageously thiostrepton or its derivatives, is used to prepare a medicament intended for the treatment of genetic muscular diseases linked to a conformational disorder of at least one protein causing its cellular degradation.

The invention thus relates to a method of treating genetic diseases linked to a conformational disorder of at least one protein, said disorder causing the cellular degradation of the protein, comprising administering to a subject in need thereof, at an efficient dose, a composition comprising a cyclic thiopeptide, advantageously thiostrepton or its derivatives.

In the frame of the invention, a cyclic thiopeptide is defined as a sulfur (S)-rich peptide having in its structure at least one cyclic ring. According to one embodiment, it has sulfur-containing heterocyclic rings, especially thiazole groups. According to another embodiment, it is further characterized by a pyridine/tetrapyridine/dehydropiperidine ring with up to three thiazole substituents on positions 2, 3 and 6. According to a specific embodiment, it has a pyridine nucleus and a macrocyclic loop connecting thiazole rings at position 2 and 3, described as ring A. According to another specific embodiment, it has a tetrahydropyridine nucleus and a quinaldic acid macrocycle connected to thiazole at position 2, described as ring B. According to a preferred embodiment, a cyclic thiopeptide to be used in the frame of the invention has a ring A and a ring B as defined above.

Also encompassed by the present invention are the salts of said peptides, e.g. sodium salts thereof.

As retrieved in the THIOBASE (http://db-mml.sjtu.edu.cn/THIOBASE/index.php), the cyclic thiopeptide can be chosen in the following list which is however not limiting:
- 68-1147 (or Sch 18640);
- A10255 B, E, G or J;
- Amythiamicin A, B, C or D;
- Beminamycin A, B, C or D;
- Cyclothiazomycin B1 or B2;
- GE2270 A, B1, B2, C1, C2a, C2b, D1, D2, E or T;
- GE37468 (A);
- Geninthiocin;
- Val-geninthiocin;
- Glycothiohexide α;
- O-methyl-glycothiohexide α;
- JBIR-83 or 84;
- Methylsulfomycin;
- Micrococcin P1 or P2;
- MJ347-81F4 A or B;
- Multhiomycin (or Nosiheptide);
- Neobeminamycin;
- Nocardithiocin;
- Nocathiacin I, II, III or IV;
- Promoinducin (Promothiocin A or B);
- QN3323 A, B or Y;
- Radamycin;
- S-54832 (A-I);
- Sch 40832;
- Siomycin A, B, C or D;
- Sulfomycin I, II or III;
- Thiostrepton (or thiactin or bryamycin or A-8506) A or B;
- Thioactin;
- Thiocillin I, II, III or IV;
- Thiomuracin A, B, C, D, E, F, G, H and I;
- Thiopeptin A1a, A1b, A3a, A3b, A4a, A4b, Ba or Bb;
- Thiotipin;
- Thioxamycin;
- TP-1161;
- YM-266183 or 84.

According to a preferred embodiment, it is chosen in the following list: 68-1147 (or Sch 18640); Cyclothiazomycin B1 or B2; Glycothiohexide α; O-methyl-glycothiohexide α; MJ347-81F4 A or B; Multhiomycin (or Nosiheptide); Nocathiacin I, II, III or IV; S-54832 (A-I); Sch 40832; Siomycin A, B, C or D; Thiostrepton (or thiactin or bryamycin or A-8506) A or B; Thiopeptin A1a, A1b, A3a, A3b, A4a, A4b, Ba or Bb.

Advantageously, the compound used in the frame of the invention is chosen in the following list: Thiostrepton A, Thiostrepton B, Siomycin A, Siomycin B, Siomycin C and Siomycin D.

Syomicin A (CAS number: 12656-09-6P), B (CAS number: 61419-19-0P), C (CAS number: 75524-62-8P) and D (CAS number: 61419-61-2) have the following formula:

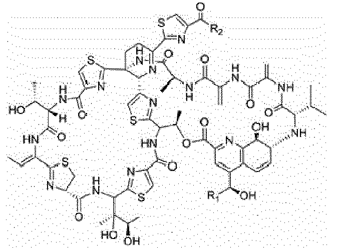

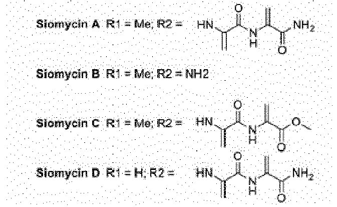

More advantageously, the compound used in the frame of the invention is thiostrepton (THSP), a salt or a derivative thereof. In other words, a composition of the invention comprises thiostrepton (THSP), a salt or a derivative thereof, as an active ingredient.

Thiostrepton is a natural cyclic oligopeptide antibiotic of the thiopeptide class, derived from several strains of streptomycetes, such as *Streptomyces azureus* and *Streptomyces laurentii.* Thiostrepton is a natural product of the ribosomally synthesized and post-translationally modified peptide (RiPP) class. It has the following formula:

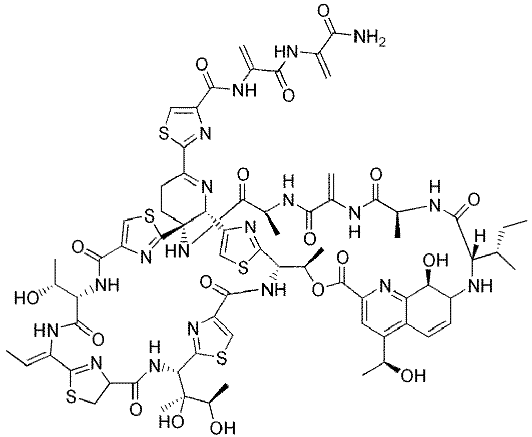

This formula in fact corresponds to thiostrepton A but a derivative thereof, thiostrepton B, is also available:

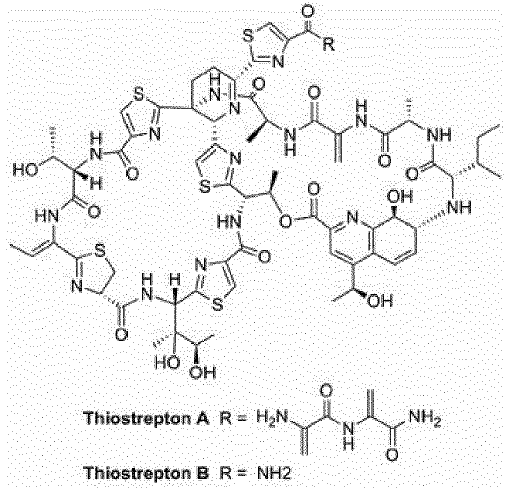

According to a specific embodiment, the cyclic thiopeptide to be used is thiostrepton A (CAS number: 1393-48-2) or thiostrepton B (CAS number: 75524-61-7), advantageously thiostrepton A.

Thiostrepton can be purified from natural sources, or obtained by biosynthesis (see e.g. Kelly and Pan, J. Am. Chem. Soc. 2009, 131(12):4327-34), or chemically synthesized using known protocols as e.g. previously disclosed by Nicolaou (Angew Chem Int Ed Engl. 2012, 51(50):12414-36). As a non-limiting example, thiostrepton provided in the form of a powder can be purchased from Sigma-Aldrich and then suspended in an adequate solvent, e. g. DMSO (Dimethyl sulfoxide).

Alternatively, a nucleotide sequence encoding the corresponding prepeptide (IASASCTTCICTCSCSS: SEQ ID NO: 12) can be introduced in a host cell for its heterologous production, e.g. in bacteria. The post-translational modifications can be performed by the host cell itself or by chemical means or *in vitro* enzymatic processes. The host cell can be further transformed with the genes encoding the enzymes involved in the required post-translational modifications.

Moreover, derivatives of thiostrepton or siomycin, including fragments and mutants thereof, can be used in the frame of the invention as long as they retain a substantial proteasome inhibition activity. Such an activity can be evaluated by different methods known to the skilled person, e.g.:
- by using the 20S Proteasome Activity Assay (Millipore) according to the instruction of the manufacturer;
- by evaluating the chymotrypsin-like activity as disclosed in the examples.

According to Sandu et al. (J. Cell. Mol. Med., Vol 19, No 9, 2015, pp 2181-92), thiostrepton is able to prevent protein degradation through the proteasome pathway, by interacting with the regulatory 19S subunit of the proteasome complex, especially with the Rpt subunits thereof, more specifically through disulfide bridges with their cysteine residues. According to another embodiment, the derivatives to be used in the frame of the invention can be tested for their ability to covalently bind to the 19S proteasome, advantageously to the Rpt subunits thereof, more advantageously through disulfide bridges with their cysteine residues, as disclosed in Sandu *et al.* Therefore, the present application proposes to use inhibitors of the 19S proteasome in that context.

For example, derivatives or analogues of thiostrepton encompassed by the invention are those reported by Key and Miller (J. Am. Chem. Soc. 2017, 139, 15460-66), wherein the subterminal dehydroalanines (Dha16 and Dha17) have been functionalized with different groups.

With regard to the compound provided in the pharmaceutical composition, especially thiostrepton, it can be the native peptide purified from an organism that naturally produces this peptide, or a recombinant peptide, possibly modified to get a derivative thereof. As already stated, a recombinant peptide according to the invention can be chemically synthesized or produced from a cellular, acellular or other synthesis system.

Said compound can be further modified to increase its stability, its bioavailability and/or its ability to reach the target tissues.

According to one embodiment, the ability of the peptide to cross the intestinal barrier can be increased by using a vector such as nanoparticles, exosomes, vesicles or cells, as known by the skilled person. As an illustration, Wang and Gartel (Mol. Cancer Ther. 2011, 10(12), 2287-97) have reported the nanoparticle encapsulation of thiostrepton: it was encapsulated into micelles assembled from amphiphilic lipid-polyethylene glycol (DSPE-PEG₂₀₀₀-MeO).

In the presence of peptides such as thiostrepton, the pharmaceutical composition can contain a nucleic acid sequence encoding said peptide and possibly the nucleic acid sequences encoding the required enzymes for its post-translational modifications. It may be present in a naked form (free) or contained in delivery systems which increase the stability, the targeting and/or the biodisponibility, such as liposomes, or incorporated into carriers such as hydrogels, cyclodextrins, biodegradable nanocapsules, bioadhesive microspheres, vectors or in combination with a cationic peptide.

According to a specific embodiment, the nucleic acid sequence is harbored by an expression system, preferably under the control of a promoter in a vector. After introduction into the body, the peptide is produced *in vivo.* The transfer of nucleic acids (DNA or RNA) can be achieved either with viral approaches to gene transfer (e.g. adeno-associated viruses or AAVs), or with non-viral approaches (e.g. by injection of a plasmid).

As already mentioned and in relation to this specific embodiment, the nucleotide sequences encoding part or all the enzymes involved in the conversion of the prepeptide to the peptide, e.g. thiostrepton, can also be provided.

The present invention also concerns pharmaceutical compositions containing as an active ingredient at least a compound as defined above, as well as the use of this compound or composition as a medicinal product or medicament.

The present invention then provides pharmaceutical compositions comprising a compound according to the invention. Advantageously, such compositions comprise a therapeutically effective amount of said compound, and a pharmaceutically acceptable carrier. In a specific embodiment, the term "pharmaceutically acceptable" means approved by a regulatory agency of the Federal or a state government or listed in the U.S. or European Pharmacopeia or other generally recognized pharmacopeia for use in animals, and humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsions, sustained-release formulations and the like. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the subject.

In a preferred embodiment, the composition is formulated in accordance with routine procedures as a pharmaceutical composition adapted for e.g. intramuscular or intravenous administration to human beings. Typically, compositions for intravenous administration are solutions in sterile isotonic aqueous buffer. Where necessary, the composition may also include a solubilizing agent and a local anesthetic such as lidocaine to release pain at the site of the injection.

In one embodiment, the composition according to the invention is suitable for administration in humans. The composition is preferably in a liquid form, advantageously a saline composition, more advantageously a phosphate buffered saline (PBS) composition or a Ringer-Lactate solution.

The amount of the therapeutic agent of the invention, i.e. a compound as disclosed above, which will be effective in the treatment of a disease can be determined by standard clinical techniques. In addition, *in vivo* and/or *in vitro* assays may optionally be employed to help predict optimal dosage ranges. The precise dose to be employed in the formulation will also depend on the route of administration, the weight and the seriousness of the disease, and should be decided according to the judgment of the practitioner and each patient's circumstances.

Suitable administration should allow the delivery of a therapeutically effective amount of the therapeutic product to the target tissues, depending on the disease.

Available routes of administration are topical (local), enteral (system-wide effect, but delivered through the gastrointestinal (GI) tract), or parenteral (systemic action, but delivered by routes other than the GI tract). In the specific case of muscular diseases, the preferred route of administration of the compositions disclosed herein is generally parenteral which includes intramuscular administration (i.e. into the muscle) and systemic administration (i.e. into the circulating system). In this context, the term "injection" (or "perfusion" or "infusion") encompasses intravascular, in particular intravenous (IV), and intramuscular (IM) administration. Injections are usually performed using syringes or catheters. According to a specific embodiment and in order to promote a localised therapeutic effect, specific muscular administration methods are preferred, particularly intramuscular injection. Conversely, in order to effectively reach all of the target tissues, systemic administration may be preferred. The so-called loco-regional administration (intravascular under pressure) is also possible.

According to one embodiment, the composition is administered orally, intramuscularly, intraperitoneally, subcutaneously, topically, locally, or intravascularly, advantageously intravenously. According to another embodiment, the composition is for intramuscular or topical administration.

As already stated, the patient is advantageously a human, particularly a new born, a young child, a child, an adolescent or an adult. The therapeutic tool according to the invention, however, may be adapted and useful for the treatment of other animals, particularly pigs, mice, dogs or macaque monkeys.

As already mentioned, the present invention relates to the treatment of genetic diseases linked to a conformational anomaly of at least one protein causing the cellular degradation thereof.

Genetic diseases are, by definition, diseases resulting from one or a plurality of mutations in one or a plurality of genes. Advantageously, the present application aims at monogenic diseases, that is, diseases linked to a single gene.

The mutations responsible for the conformational disorder of the resulting protein may be point mutations. However, the conformational disorder may be linked to mutations which are larger than points, for example, the deletion of a codon in the gene which codes a protein which is still active if it is not degraded.

In the context of the invention, "conformational disorder of at least one protein" or "protein conformational disorder" means the fact that the protein causing the disease is misfolded, due to the presence of at least one mutation in the gene encoding it, said disorder causing the degradation of said protein by the cell, especially by the proteasome pathway. The targeted pathologies can thus be easily identified, for example, by means of antibodies directed against the mutated protein. Indeed, even if it is correctly expressed (which may be verified at the transcript level), it is poorly detected by means of such an antibody, since it is at least partially degraded.

In the context of the invention, the provided solution relies on the use of cyclic thiopeptides, advantageously thiostrepton or its derivatives, to avoid the degradation of mutated proteins, to ensure their addressing in the final cellular compartment, and thus to restore a normal phenotype.

Thiostrepton and siomycin have already been proposed as a drug for the treatment of cancers based on their ability to inhibit the oncogenic transcription factor FoxM1 and to stabilize proteins like p53 via proteasome inhibition. Cancer is clearly out of the definition of the diseases to be treated in the frame of the present invention.

According to a specific aspect, the protein having the conformational disorder and submitted to the cellular degradation is a protein of the membrane or associated with the membrane, possibly integrated in a protein complex.

As illustrated in the present application, the present invention is useful for the treatment of muscle pathologies, advantageously those affecting the skeletal muscles but also possibly the heart muscle.

According to a specific embodiment, the present invention aims at recessive muscular pathologies.

Advantageously, the genetic muscular disease linked to a protein conformational disorder is a muscular dystrophy, more advantageously a progressive muscular dystrophy of proximal as well as of distal type.

Among progressive muscular dystrophies, the following diseases may particularly be mentioned:
- sarcoglycanopathies, that is, α-sarcoglycanopathy or LGMD2D linked to an α-sarcoglycan defect, β-sarcoglycanopathy or LGMD2E linked to a β-sarcoglycan defect, γ-sarcoglycanopathy or LGMD2C linked to a γ-sarcoglycan defect, or δ-sarcoglycanopathy or LGMD2F linked to a δ-sarcoglycan defect;
- dysferlinopathies (LGMD2B or Miyoshi myopathy) linked to a dysferlin defect;
- anoctaminopathies (LGMD2L or Miyoshi type 3 myopathy) linked to an anoctamin 5 defect;
- Limb girdle myopathy with a FKRP or LGMD2I, linked to a defect of the FKRP ("fukutin-related protein").

More generally, the targeted diseases are selected from the following group:
- progressive muscular dystrophies:
   ∘ implying dysferlin (DYSF)
   ∘ implying γ-sarcoglycan
   ∘ implying α-sarcoglycan
   ∘ implying β-sarcoglycan
   ∘ implying δ-sarcoglycan
   ∘ implying anoctamin 5 (Ano5)
   ∘ implying the fukutin related protein (FKRP)
   ∘ implying fukutin (FKTN)
   ∘ implying protein-O-mannosyltransferase 1 (POMT1)
   ∘ implying protein-O-mannosyltransferase 2 (POMT2)
   ∘ implying protein-O-linked mannose β 1,2-acetylglucosaminyl-transferase (POMGT1)
   ∘ implying caveolin 3 (Cav3)
   ∘ implying UDP-N-acetyl glucosamine 2-epimerase (GNE)
- congenital muscular dystrophies (CMD):
   ∘ implying α-dystroglycan (DAG1)
   ∘ implying laminin alpha-2 (LAMA2)
   ∘ implying like-glycosyltransferase (LARGE)
   ∘ implying collagen 6A1, 6A2, or 6A3
   ∘ implying selenoprotein 1 (SEPN1)
   ∘ implying integrin alpha7 (ITGA7)
   ∘ the ryanodine receptor (RYR)
- other diseases affecting the skeletal or heart muscle, possibly associated with impairments of other organs:
   ∘ arrhythmogenic right ventricular cardiomyopathy implying transmembrane protein 43 (TMEM43)
   ∘ type 4 liposdystrophy muscular dystrophy implying polymerase I and the transcript release factor (PTRF)
   ∘ the chondrodystrophic myotonia or Schwartz Jampel Syndrome implying heparan sulfate (HSPG2)
   ∘ the Danon disease implying the lysosomal-associated membrane protein 2 (LAMP2)
   ∘ Fibrodysplasia ossificans progressiva implying the type I activin A receptor (ACVR1).

For a same disease, different mutations may affect the same gene and be responsible for the conformational disorder of the encoded protein. Thus, and as examples for sarcoglycanopathies, the main point mutations listed to date are listed in document WO2008/009802.

In the context of the present application, the feasibility of the invention has for example been demonstrated in relation with the R77C, R34H, I124T and V247M mutations of α-sarcoglycan.

According to another embodiment, the present invention is useful for the treatment of skin diseases. A non-limiting list of such diseases includes:
- Junctional Epidermolysis Bullosa linked to a COL17A1 defect;
- Lamellar Ichthyosis linked to a Transglutaminase 1 Gene (TGM1) defect;
- Disseminated superficial actinic porokeratosis linked to a Mevalonate Kinase gene (MVK) defect;
- Recessive cutis laxa linked to a fibulin-5 defect;
- Systemic Hyalinosis linked to a CMG2 (capillary morphogenesis gene 2) defect;
- Epidermolysis bullosa linked to a Plectin defect;
- Junctional epidermolysis bullosa linked to a Laminin defect;
- Pustular Psoriasis linked to an Interleukin 36 receptor antagonist (IL-36Ra) defect;
- Autosomal Recessive Congenital Ichthyosis linked to a Type I Transglutaminase (TG1) defect;
- Congenital erythropoietic porphyria (CEP) linked to an uroporphyrinogen III synthase (UROS) defect;
- Tyrosinase-negative albinism linked to a Tyrosinase defect;
- Junctional epidermolysis bullosa with pyloric atresia linked to an Integrin b4 (ITGB4) defect.

According to a further embodiment, the present invention is useful for the treatment of diseases linked to mutations in the cystic fibrosis transmembrane conductor regulator (CFTR), e.g. the ΔF508 mutation, such as the treatment of cystic fibrosis.

According to one aspect, the composition according to the invention is associated with other treatments for the same disease.

According to a specific embodiment, the present invention concerns a composition, advantageously a pharmaceutical composition or a medicinal product containing a compound as described above and potentially other active molecules (other gene therapy proteins, chemical groups, peptides or proteins, etc.) for the treatment of the same disease or a different disease, advantageously of the same disease.

In relation to muscular dystrophies, simultaneous or sequential administration of peptides or proteins able to increase the muscle mass, such as decorin (WO2010/106295) or fibromodulin (WO2013/072587), can be envisaged.

More generally, in relation to genetic disease linked to a conformational disorder of at least one protein, a further compound able to prevent the cellular degradation of the protein can be administered simultaneously or at different times. In case of simultaneous administration, the two compounds can be associated in the same composition.

In that context, various compounds can be used, e.g.:
- mannosidase I inhibitors as disclosed in Bartoli, M. *et al.* (2008), in particular kifunensine;
- proteasome inhibitors as disclosed in Gastaldello, S. *et al.* (2008), in particular MG132 and bortezomib;
- epigenome modulators as disclosed in WO2014/013184, in particular phenylbutyrate, SAHA and 5-aza.

According to a preferred embodiment and as supported by the present application showing a synergy between thiostrepton and bortezomib, the compound of the invention is combined with a further proteasome inhibitor. Such a proteasome inhibitor is advantageously chosen in the following list: bortezomib (or Velcade or PS-341), MG115, MG132, MG262, MG110, lactacystin, epoxomicin, eponemycin, carfilzomib, CEP-18770, MLN2238, ONX-0912, marizomib, omuralide, more advantageously bortezomib or MG132.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques are applicable to the production of the polynucleotides and polypeptides of the invention, and, as such, may be considered in making and practicing the invention. Particularly useful techniques for particular embodiments will be discussed in the sections that follow.

The disclosures of each and every patent, patent application, and publication cited herein are hereby incorporated herein by reference in their entirety.

Without further description, it is believed that one of ordinary skill in the art can, using the preceding description and the following illustrative examples, make and utilize the compounds of the present invention and practice the claimed methods.

### EXAMPLES

The invention and its advantages are understood better from the examples shown below supporting the annexed figures. In particular the present invention is illustrated with regard to the effect of thiostrepton in an *in vitro* model for LGMD2D, linked to mutations (R77C, R34H, I124T and V247M substitutions) in the gene encoding α-sarcoglycan (α-SG). These examples are not however in any way limiting.

### BRIEF DESCRIPTION OF THE DRAWINGS

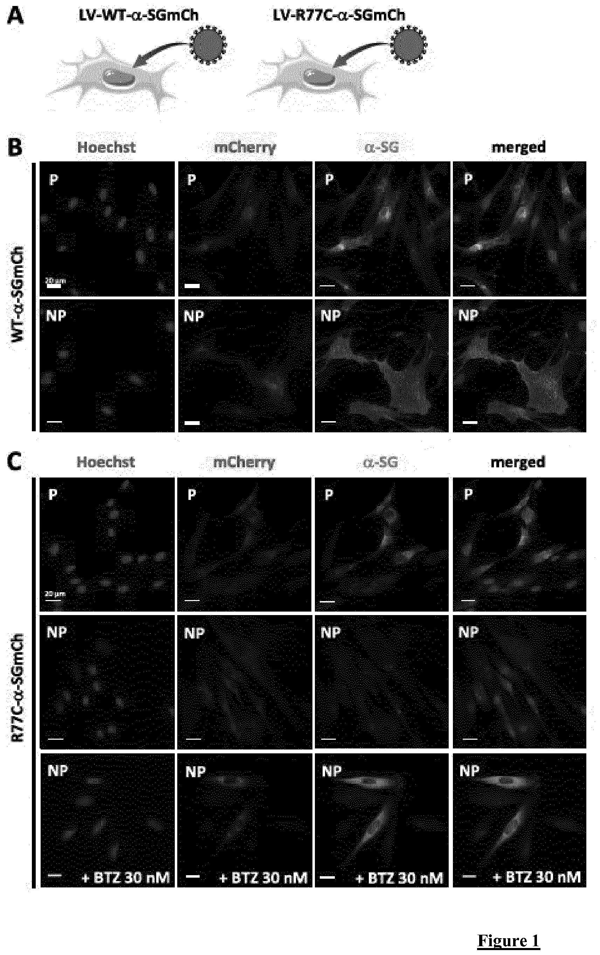

**Figure 1****: Characterization of α-sarcoglycan WT and R77C fusion constructs and effect of bortezomib treatment.**
   (A) Schematic representation of the cellular models showing that immortalized fibroblasts from a LGMD2D patient carrying the R77C homozygous mutation were transduced with lentivirus expressing WT-α-SGmCh or R77C-α-SGmCh constructs.
   (**B**) Confocal images of mCherry signal and α-SG detected by immunofluorescence in fibroblasts transduced with the lentivirus expressing WT-α-SGmCh under permeabilized (P) and non-permeabilized condition (NP).
   (**C**) Confocal images of mCherry signal and α-SG detected by immunofluorescence in fibroblasts transduced with the lentivirus expressing R77C-α-SGmCh under permeabilized (P) and non-permeabilized condition (NP) and following bortezomib (BTZ) treatment at 30 nM. Nuclei are labelled by Hoechst staining. Scale bar = 20 µm.

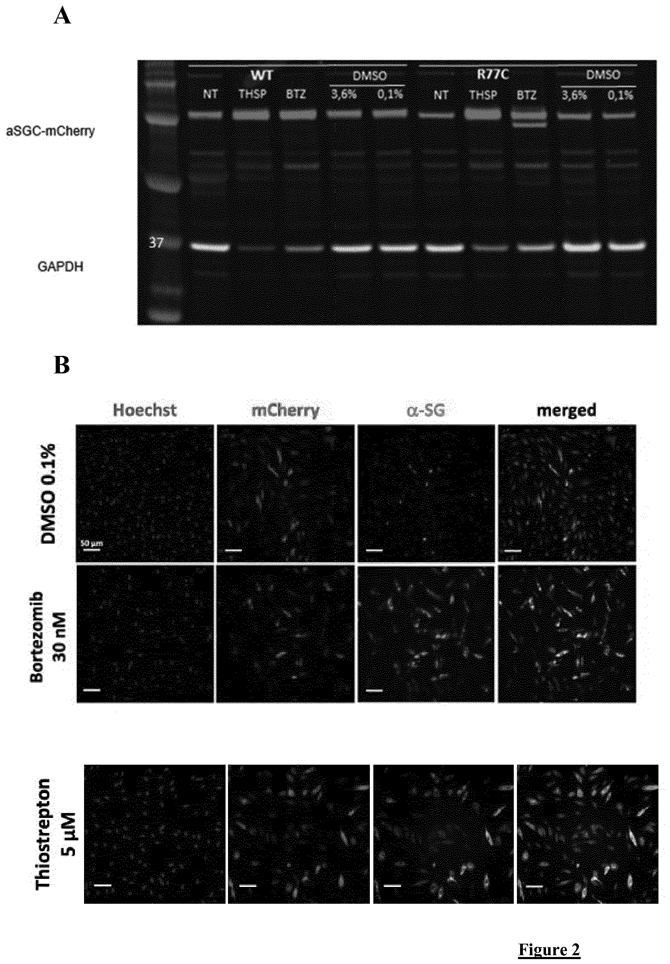

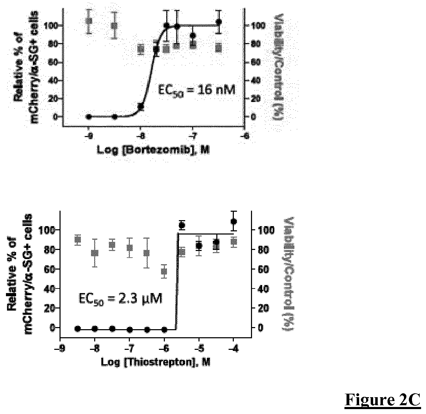

**Figure 2****: Effect of thiostrepton on expression and membrane localization of R77C α-SG protein.**
   (**A**) mCherry fluorescent signal (red) in fibroblasts overexpressing WT-α-SGmCh or R77C-α-SGmCh not treated (NT), or treated with bortezomib 30 nM (BTZ) in DMSO (3.6%) or thiostrepton 3 µM (THSP) in DMSO (0.1%). GAPDH was used for normalization (green).
   (**B**) mCherry fluorescent signal and α-SG staining in non permeabilized condition in fibroblasts overexpressing R77C-α-SGmCh treated with 0.1% DMSO, boretezomib 30 nM and thiostrepton at the dose of 5 µM. Nuclei are labelled by Hoechst staining. Scale bar = 50 µm.
   (**C**) Quantification of mCherry and membrane α-SG positive cells and cell viability following treatment with increasing concentrations of bortezomib and thiostrepton. Values are expressed as the percentage of the maximal response induced by bortezomib and each point represents the mean±SD of four replicates; thiostrepton = THSP; bortezomib= BTZ.

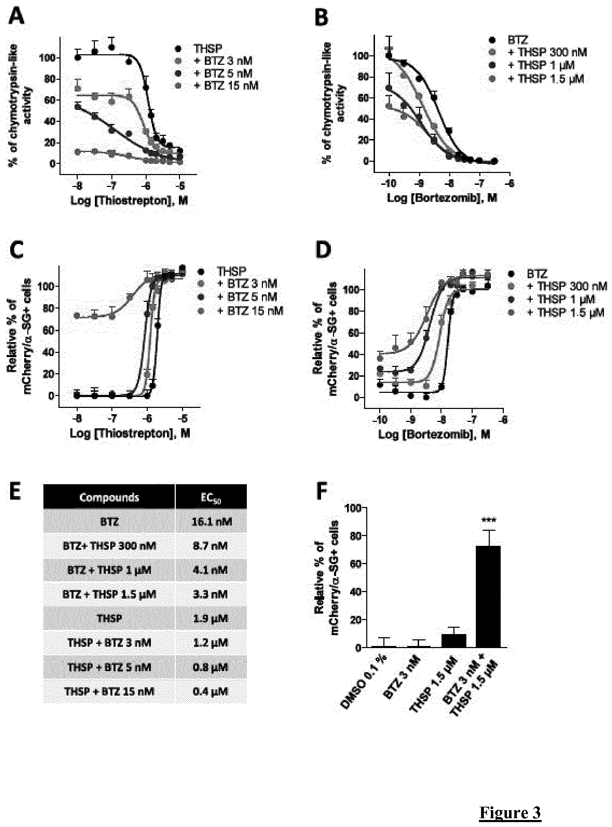

**Figure 3****: Synergistic effects of thiostrepton and bortezomib.**
   Quantification of chymotrypsine-like activity of the proteasome (**A-B**) or mCherry and membrane α-SG expression (**C-D**) in fibroblasts overexpressing R77C-α-SGmCh. Cells were treated for 24 h with increasing concentration of thiostrepton in the presence of 3 nM, 5 nM or 15 nM bortezomib (**A, C**) or with increasing concentrations of bortezomib in the presence of 300 nM, 1 µM or 1.5 µM thiostrepton (**B, D**). Values are expressed as percentage of the response induced by 0.1% DMSO (**A-B**) or as percentage of the maximal response induced by bortezomib (**C-D**) and each point represents the mean±SD of four replicates. (**E**) EC₅₀ of thiostrepton, bortezomib and combination treatments on membrane α-SGmCh membrane rescue. (**F**) Quantification of mCherry and membrane α-SG positive cells after 0.1% DMSO, 1.5 µM thiostrepton, 3 nM bortezomib and combination treatments. ***p ≤ 0.001; thiostrepton = THSP; bortezomib= BTZ.

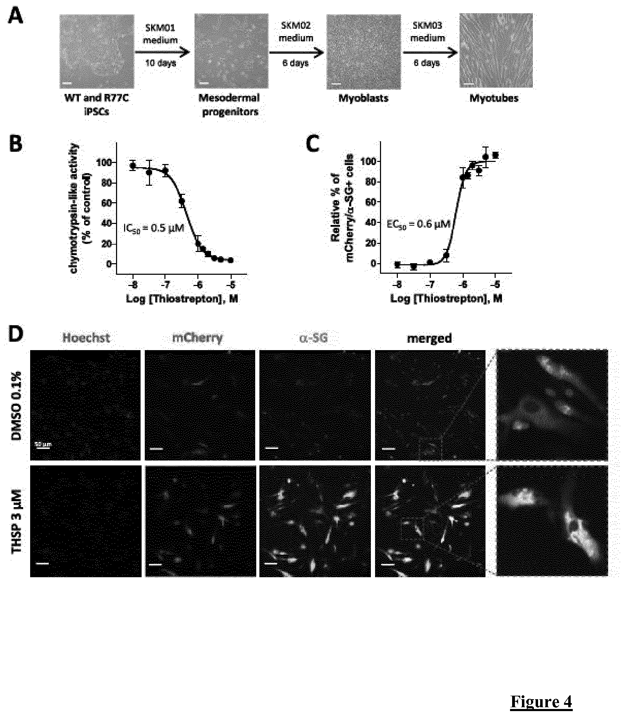

**Figure 4** **: Thiostrepton effect on the rescue of R77C-α-SGmCh protein in iPSC-derived myoblasts.**
   (**A**) Differentiation of iPSCs into myoblasts following a 3-step protocol of differentiation. iPSC-derived myoblasts were transduced with the lentivirus overexpressing R77C-α-SGmCh and treated for 24 h with increasing concentrations of thiostrepton or negative and positive controls (0.1% DMSO and 30 nM bortezomib, respectively).
   (**B**) Quantification of the chymotrypsine-like activity of the proteasome following thiostrepton 3µM treatment. Values are expressed as the percentage of the response induced by DMSO (0.1%).
   (**C**) Quantification of mCherry and membrane α-SG positive cells after thiostrepton 3µM treatment. Values are expressed as percentage of the maximal response induced by bortezomib.
   (**D**) Images of mCherry fluorescent signal, membrane α-SG and Hoechst staining in iPSC-derived myoblasts treated with 0.1% DMSO or thiostrepton 3 µM in non-permeabilized condition. Outsets represent higher magnification images. Scale bar = 50 µm; thiostrepton = THSP; bortezomib= BTZ.

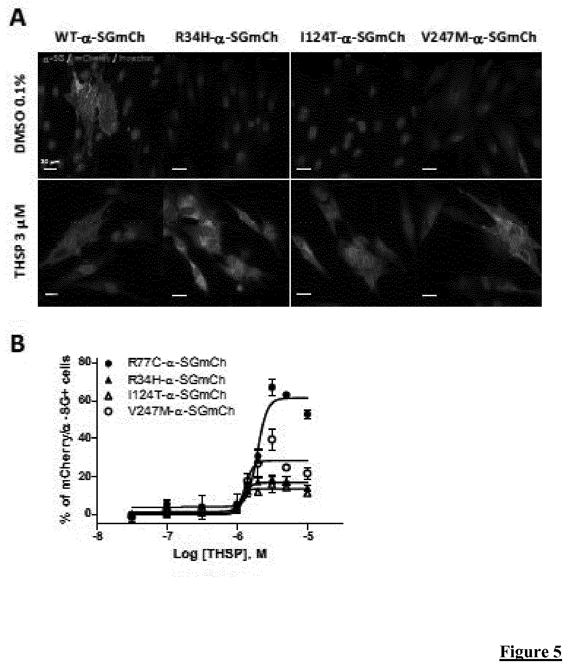

**Figure 5** **: Evaluation of thiostrepton treatment on other missense α-SG mutants.** Immortalized fibroblasts were transduced with lentivirus expressing WT-, R34H-, I124Tor V247M-α-SGmCh constructs and treated for 24 h with 0.1% DMSO or increasing concentrations of thiostrepton. The membrane α-SG expression was monitored by IF under non-permeabilized condition.
   (**A**) Confocal images of mCherry fluorescent signal, membrane α-SG (green) and Hoechst staining after 0.1% DMSO or THSP 3 µM treatments. Scale bar is 20 µm.
   (**B**) Quantification of mCherry and membrane α-SG positive cells following treatment with increasing concentrations of Thiostrepton. Values are expressed as percentage of mCherry and α-SG positive cells; thiostrepton = THSP; bortezomib= BTZ.

### MATERIALS AND METHODS

### Plasmid cloning and mutagenesis.

The sarcoglycan fusion protein was designed based on the human *SGCA* consensus coding sequence (CCDS) found in the NCBI portal (Gene ID: 6442, CCDS number 45729.1) and the mCherry sequence: The linker, in the amino-acid form of -GGGGS-, was chosen as a flexible type linker that also increased stability/folding of the fusion proteins. The fusion protein nucleotide sequence was synthesized by Genecust and cloned in a vector plasmid prepared for lentivirus production. The final construct, termed α-SGmCh, was driven by the cytomegalovirus (CMV) promoter. The R34H, R77C, I124T and V247M mutations were generated based on the 100GT > AC, 229C > T, 371T > C and 739G > A nucleotide changes of *SGCA* gene, respectively. Mutagenesis was performed using the QuikChange XL Site-Directed Mutagenesis kit (200516, Agilent) according to manufacturer's instructions.

The primers used to introduce the R34H mutation were:
Forward 5'-CACCCACTTGTGGGCCACGTCTTTGTGCACACC-3' (SEQ ID NO: 1) and
Reverse 5'-GGTGTGCACAAAGACGTGGCCCACAAGTGGGTG-3' (SEQ ID NO: 2), for R77C mutation:
Forward 5'-GCCCCGGTGGCTCTGCTACACCCAGCGC-3' (SEQ ID NO: 3) and
Reverse 5'-GCGCTGGGTGTAGCAGAGCCACCGGGGC-3' (SEQ ID NO: 4), for I124T mutation:
Forward 5'-CTGGTGCTGGAGACTGGGGACCCAGAA-3' (SEQ ID NO: 5) and
Reverse 5'-TTCTGGGTCCCCAGTCTCCAGCACCAG-3' (SEQ ID NO: 6) and for V247M mutation:
Forward 5'-GTTGACTGGTGCAATATGACCCTGGTGGATA-3' (SEQ ID NO: 7) and
Reverse 5'-TATCCACCAGGGTCATATTGCACCAGTCAAC-3' (SEQ ID NO: 8).

### Cell Culture, transduction and pharmacological treatments.

The R77C fibroblasts used in this study were isolated from a patient biopsy obtained by the Genethon's Cell Bank. Informed consents were obtained from the parents of the patient included in this study, complying with the ethical guidelines of the institutions involved and with the legislation requirements of the country of origin.

Fibroblasts were transduced with a pBABE-puro-based retroviral vector containing sequence encoding the catalytic subunit of human telomerase reverse transcriptase (hTERT) and then selected in the presence of puromycin (1 mg/ml) for 10 days, as previously described (Chaouch, S. et, Human gene therapy 20, 784-790 (2009)). Fibroblasts were cultured in Dulbecco's modified Eagle's medium+GlutaMAX (Invitrogen) supplemented with 10% fetal bovine serum (research grade, Sigma-Aldrich) and 1% Penicillin-Streptomycin (Invitrogen).

For overexpression of the WT or mutated α-SG, immortalized fibroblasts were transduced with a lentiviral vector expressing human WT-, R77C-, R34H-, I124T- or V247M-α-SGmCh with a multiplicity of infection (MOI) of 20 in the presence of 4 µg/ml of polybrene (Sigma-Aldrich). Cells were seeded on plates coated with 50µg/ml of collagen I and maintained in a humidified atmosphere of 5% CO2 at 37°C. Twenty-four hours after seeding, cells were treated with thiostrepton (Sigma Aldrich), bortezomib (Selleckchem) or the carrier dimethyl sulfoxide (DMSO, VWR). Cells were analyzed after 24 hours of treatment.

### Pluripotent stem cell culture and differentiation.

The R77C fibroblasts were reprogrammed to iPSCs using Yamanaka's original method with OCT4, KLF4, SOX2, c-Myc, transferred using retroviral vectors (Takahashi, K. & Yamanaka, S., Cell 126, 663-676 (2006)). WT and R77C iPSCs were grown in colonies on matrigel (Corning), seeded at 5000 cells/cm² and cultured in iPS-Brew XF medium (Macs, Milteny Biotech). iPSCs were differentiated into myoblasts following a protocol developed by Geneabiocell® (Caron et al, 2016). Briefly, iPSCs were seeded at 3,500 cell/cm² in 6-well plates coated with collagen I and maintained in SKM01 medium (AMSBIO) for 10 days with a passage at day 7. Medium was then switched to SKM02 medium (AMSBIO) until freezing of the myoblasts at day 16. For overexpression of the WT or mutated α-SG, iPSC-derived myoblasts were transduced with a lentiviral vector expressing human WT or R77C-α-SGmCh with a multiplicity of infection (MOI) of 43 in the presence of 4 µg/ml of polybrene (Sigma-Aldrich).

### Immunoblotting.

Twenty-four hours after seeding, the immortalized fibroblasts expressing the WT and R77C sarcoglycan were treated with thiostrepton 3uM (Sigma Aldrich), bortezomib 30nM (Selleckchem) or the carrier dimethyl sulfoxide 0.1% (DMSO, VWR). Cells were collected after 24 hours of treatment. Proteins were extracted by cell lysis buffer (RIPA Buffer, Thermo Scientific) and Proteases Inhibitors (Complete PIC, Roche) and benzonase 1:10 000 (Millipore). Proteins were separated using a NuPAGE pre-cast 4-12% Bis-Tris gel and then transferred to nitrocellulose membrane with iBlot2 Dry Blotting system (ThermoFisher) using the 8 min30/20V program. Detection of proteins was performed using standard Odyssey protocol by incubation with anti-alpha sarcoglycan monoclonal (Novocastra, ref: NCL-L-aSARC, dilution 1/200) and anti-GAPDH polyclonal Rabbit (FL-335, Santa Cruz, dilution 1/500) antibodies. Western blot were revealed using Odyssey secondary antibodies donkey anti mouse (DAM) 680, and donkey anti rabbit (DAR) 800 diluted 1:5000, 1H30 at RT and then scanned with the Odyssey machine.

### α-SG localization cell-based assay.

After 24 hours of drug treatment, cells were fixed in 4% paraformaldehyde (10 min, room temperature). Immunocytochemistry was performed in a phosphate-buffered saline (PBS) solution supplemented with 1% bovine serum albumin (BSA; Sigma) for blocking (1 hour, room temperature) and with a mouse anti-α-SG (NCL-L-a-SARC, Novocastra, Leica) for primary hybridization step (overnight, 4 °C). Cells were stained with a fluorophore-conjugated secondary anti-mouse antibody (Invitrogen; 1 hour, room temperature) and nuclei were visualized with Hoechst 33342 (Invitrogen). α-SG localization was analyzed with a CellInsight CX7 HCS Platform (Cellomics Inc). The first channel was used for nuclei identification, the second one for membrane α-SG staining identification and the third one for mCherry tag identification. Pictures were acquired with a 10x objective in high-resolution camera mode and were analyzed using the colocalization bioapplication.

### Data analysis.

Data analysis was performed using a customized Hiscreen application (Discngine) connected to Spotfire software (Tibco Software Inc.). The robustness of the assay was evaluated by calculating for each plate the Z' factor on the percentage of mCherry/α-SG positive cells parameter as follows Z'=1-[3(SDP+SDN)/(MP- MN)] where MP and MN correspond to the means of the positive (30 nM bortezomib) and negative (0.1% DMSO) controls, respectively, and SDP and SDN correspond to their S.D. Raw data related to the percentage of mCherry/α-SG positive cells were normalized to the mean of positive and negative controls, which are defined as 100% and 0%, respectively. Raw data related to cell number per field were normalized to the mean of negative controls. Hit selection was performed using in parallel the number of standard deviations from the mean for each readout value (Z-score) calculated per plate individually or per run where all plate data were pooled. Only hits whose Z-score plate and/or Z-score run was ≥3 and that did not decrease cell number by more than 55% compared to 0.1% DMSO condition were selected for subsequent validation step. Hits were then tested at gradual concentrations (1 nM - 100 µM) for parallel exploration of their efficacy and toxicity.

### Quantitative PCR.

Total RNAs were isolated using the RNeasy Mini extraction kit (Qiagen) according to the manufacturer's protocol. A DNase I digestion was performed to avoid genomic DNA amplification. RNA levels and quality were checked using the NanoDrop technology. A total of 500 ng of RNA was used for reverse transcription using the SuperScript III reverse transcription kit (Invitrogen). Quantitative polymerase chain reaction (qPCR) analysis was performed using a QuantStudio 12 K Flex real-time PCR system (Applied biosystem) and Luminaris Probe qPCR Master Mix (Thermo Scientific), following the manufacturers' instructions. α-SG expression analysis were performed using the TaqMan gene expression Master Mix (Roche), following the manufacturer's protocol. Quantification of gene expression was based on the DeltaCt method and normalized on 18S expression (Assay HS_099999).
Primer sequences for *SGCA* were :
   5'-TGCTGGCCTATGTCATGTGC-3' (SEQ ID NO: 9) and
   5'-TCTGGATGTCGGAGGTAGCC-3' (SEQ ID NO: 10).
Taqman MGB probe sequence for SGCA was:
   5'-CGGGAGGGAAGGCTGAAGAGAGACC-3' (SEQ ID NO: 11).

### Proteasomal activity assay.

Fibroblasts and myoblasts were seeded in 384 well plates and treated with various concentrations of bortezomib (100 pM - 300 nM) and thiostrepton (10 nM - 10 µM) for 12 hours. Proteasome-Glo™ or chymotrypsin-like cell-based assay reagents were added according to manufacturer instructions (Promega). Luminescence was read using a CLARIOstar® microplate reader (BMG Labtech).

### Statistical analysis.

Data are presented as means ± SD. Statistical analysis was performed using the Student's t test. Statistical significance was considered for ***P≤0.001. Curve-fitting, IC₅₀ and EC₅₀ determinations were performed using GraphPad Prism (v5.0.3).

### RESULTS

### 1/ Validation of the α-SGmCh fusion construct and characterization of the R77C mutant cellular model.

In order to easily evaluate the membrane localization of the wildtype (WT) and R77C mutant α-SG in the heterologous condition, a system was generated that allowed the simultaneous identification of the positive cells for the exogenous protein and the quantification of the amount of protein in the cell membrane. A fusion protein was created, consisting of the coding sequence for human WT or R77C-α-SG fused with an mCherry (mCh) fluorescent reporter at the C-terminus. A flexible linker was inserted between the two coding sequences to avoid protein interference in protein maturation and folding. The constructs, hereafter termed WT-α-SGmCh and R77C-α-SGmCh, were placed under the transcriptional control of the cytomegalovirus (CMV) promoter and inserted into a lentivirus backbone. Immortalized fibroblasts from a LGMD2D patient homozygous for R77C were used as cellular model because of their absence of endogenous α-SG. WT-α-SGmCh lentivirus was transduced with a multiplicity of infection (MOI) of 20 in these cells and monitored by evaluating the mCh fluorescence signal (Figure 1A and 1B, top panels). The integrity of the fused protein was confirmed by showing co-localization of the mCh signal (red) and α-SG, as detected with an α-SG antibody (NCL-L-a-SARC) directed against the extracellular domain of α-SG (green) in a permeabilized condition (Figure 1B, top panels). Immunofluorescence (IF) staining was then performed with the same α-SG antibody in a non-permeabilized condition, indicating that the fused protein was properly located at the cell membrane localization even in the absence of the other sarcoglycans as it was previously observed on other cell types (Gastaldello, S. *et al.* (2008)). A presence positive staining of α-SG was only detected in with mCh signal (Figure 1B, bottom panels), confirming that the membrane protein revealed by IF was produced by the exogenous SGCA sequence.

The R77C-α-SGmCh fusion protein was characterized after validation of the WT-α-SGmCh construct. The choice of this particular mutation was based on the number of occurrences of patients affected with this mutation that were reported in the Leiden Muscular Dystrophy database (http://www.dmd.nl) and on previous studies demonstrating the rescue of the R77C protein using pharmacological treatments (Bartoli, M. *et al.* (2008), Gastaldello, S. *et al.* (2008)). Confocal analysis of α-SG IF revealed intracellular staining in the permeabilized condition (Figure 1C, top panels) and no detectable membrane localization of the R77C mutant protein in the non-permeabilized condition (Figure 1C, middle panels), indicating retention of the protein. Further investigations of α-SG subcellular localization, with staining of the ER based on the recognition of the KDEL signal sequence, confirmed the ER retention of the R77C mutant protein (data not shown). Finally, the relevance of the model was validated by showing the rescue of R77C α-SG localization in the plasma membrane, following a pharmacological proteasome inhibition with bortezomib (BTZ) at 30 nM (Figure 1C, bottom panels).

### 2/ Identification of thiostrepton as a new inhibitor of misfolded R77C-α-SG protein degradation.

As shown in Figure 2, thiostrepton (THSP), a natural cyclic oligopeptide antibiotic approved in veterinary medicine for the topical treatment of dermatologic infections, is capable of the expression and the membrane localization of R77C-α-SGnCh, with potentially lower toxicity than the proteasome inhibitor bortezomib (BTZ), with an EC₅₀ of 2.3 µM and a toxicity of only 20%. Of note, THSP induced a steep dose-response curve, with a threshold dose of 1 µM and maximum effect at 3 µM.

### 3/ Combinatorial effect of thiostrepton and bortezomib on R77C-α-SGnCh membrane rescue.

The chymotrypsin-like activity of the proteasome was measured with increasing concentrations of THSP in the presence of 3 nM, 5 nM or 15 nM bortezomib in one hand and with increasing concentrations of bortezomib in the presence of 300 nM, 1 µM or 1.5 µM THSP in the other hand. Analysis of the dose-response curves revealed a higher inhibition of the chymotrypsin-like activity induced by THSP when combined with bortezomib than with the two drugs used alone (Figures 3A and 3B). Quantification of the number of cells with membrane localization of this protein was carried out in the presence of increasing doses of these two drugs, either alone or in combination, to determine whether or not THSP and bortezomib might act synergistically on R77C-α-SG mutant rescue. Analysis of the dose-response curves revealed a shift to the left when the drugs are combined, in comparison to the individual drugs alone (Figures 3C and 3D). Analysis of this set of experiments revealed a decrease in the EC₅₀ obtained following drug combination treatments (3.3 nM and 0.4 µM, respectively), as compared to the individual drugs alone, i.e., 16.1 nM bortezomib and 1.9 µM THSP (Figure 3E). Finally, this synergistic effect on R77C-α-SGmCh mutant protein rescue was confirmed by combining low doses of bortezomib (3 nM) and THSP (1.5 µM), showing a significantly higher effect of this combination than with the two drugs alone (Figure 3F). Overall, these results provide evidence of a synergistic effect of THSP and bortezomib on R77C-α-SGmCh mutant protein rescue from proteasomal degradation.

### 4/ Rescue of the R77C-α-SG mutant in iPSC-derived myoblasts following thiostrepton treatment.

The efficacy of THSP in rescuing the R77C-α-SGmCh mutant protein was assessed on muscular cells to confirm the data collected with the fibroblast cellular model. WT and LGMD2D patient fibroblasts were reprogrammed into induced pluripotent stem cells (iPSCs) as no primary myoblasts carrying the homozygous R77C mutation were available. Following a full quality control of their pluripotency capacities, as revealed by the expression of OCT4, NANOG, SSEA4, TRA160 by IF or SSEA3, TRA181, based on flow cytometry and a high activity of alkaline phosphatase (data not shown), iPSCs were then differentiated into myoblasts and myotubes using a 3-step protocol of differentiation (Figure 4A). Myoblasts and myotubes obtained using this protocol were characterized by monitoring the expression of the two myogenic markers, MyoD and MF20 (data not shown). While qPCR revealed high expression of *SGCA* mRNA (data not shown), the endogenous protein was not detected by IF, preventing its use for investigating the effect of THSP (data not shown). Myoblasts derived from LGMD2D patient iPSCs were therefore transduced with the lentivirus overexpressing R77C-α-SGmCh to evaluate the effect of THSP on muscular cells. Quantification of chymotrypsin-like activity in these cells revealed a dose-dependent effect of THSP, with an IC₅₀ of 0.5 µM (Figure 4B). Similarly, analysis of R77C-α-SGmCh protein localization revealed a rescue of this protein in the plasma membrane, with an EC₅₀ of 0.6 µM (Figure 4C and 4D).

### 5/ Rescue of different α-SG mutants through thiostrepton treatment.

The effect of THSP was then evaluated on the rescue of other missense α-SG mutants. We selected three mutations with severe phenotypes (R34H, I124T and V247M) that are among the most frequent mutations in *SGCA* encoding for misfolded proteins that are not rescuable by kifunensine. Lentiviruses expressing the R34H, I124T and V247M α-SGmCh mutants were generated following the same experimental strategy employed for the R77C mutant and were used to transduce the immortalized R77C patient fibroblasts. Confocal immunofluorescence analysis in non-permeabilized condition revealed the absence of detectable α-SG staining in the membrane compartment in all the mutant transduced cells (Figure 5A, top panels). Analysis of R34H, I124T and V247M-α-SGmCh protein localization was then evaluated following treatment with 3 µM THSP and showed the rescued localization of the three mutant proteins at the membrane level (Figure 5A, bottom panels). Finally, analysis of the dose-response curves revealed a similar effect of THSP, with an EC₅₀ of 1.4 µM, on R77C, I124T, R34H and V247M α-SGmCh mutant proteins, but different maximum efficacies ranging between 15% and 60% of rescued cells (Figure 5B).

### CONCLUSION

The present study identifies THSP as a candidate molecule able to rescue α-SG mutant proteins from ER-retention and early degradation and then as a new therapeutic molecule for the treatment of LGMD2D patients affected by missense mutations.

Moreover, even though the study demonstrates that bortezomib treatment can efficiently rescue R77C-α-SGmCh protein degradation, overall, it reports that the combination of bortezomib and THSP significantly increases the effect of the treatment on the proteasome activity and the α-SG rescue.

## Claims

1. A pharmaceutical composition comprising a cyclic thiopeptide for use in the treatment of a genetic disease linked to a conformational disorder of at least one protein, said disorder causing the cellular degradation of the protein.

2. A composition for its use according to claim 1, wherein the compound is thiostrepton (THSP) or siomycin, advantageously thiostrepton (THSP), a salt or a derivative thereof.

3. A composition for its use according to claim 1 or 2, wherein the compound is in the form of a peptide or a nucleic acid encoding said peptide, possibly harbored by an expression vector.

4. A composition for its use according to any of the preceding claims, wherein the genetic disease linked to a conformational disorder of at least one protein is a muscular dystrophy, a skin disease or a disease linked to a mutated cystic fibrosis transmembrane conductor regulator (CFTR) such as cystic fibrosis.

5. A composition for its use according to claim 4, wherein the muscular dystrophy is selected from the group consisting of: sarcoglycanopathies, dysferlinopathies, anoctaminopathies, and dystrophies associated with a FKRP ("Fukutin-Related Protein") disorder, advantageously a sarcoglycanopathy, more advantageously an α-sarcoglycanopathy.

6. A composition for its use according to claim 4, wherein the protein is selected from the group consisting of: sarcoglycan, advantageously α-sarcoglycan, dysferlin, anoctamin 5, Fukutin-Related Protein (FKRP).

7. A composition for its use according to claim 4, wherein the skin disease is selected from the group consisting of: junctional Epidermolysis Bullosa, Lamellar Ichthyosis, Disseminated superficial actinic porokeratosis, Recessive cutis laxa, Systemic Hyalinosis, Epidermolysis bullosa, Pustular Psoriasis, Autosomal Recessive Congenital Ichthyosis, Congenital erythropoietic porphyria (CEP), Tyrosinase-negative albinism, Junctional epidermolysis bullosa with pyloric atresia.

8. A composition for its use according to any of the preceding claims, wherein the composition is associated with other treatments for the same disease.

9. A composition for its use according to any of the preceding claims, wherein the composition comprises a further compound for treating the same disease.

10. A composition for its use according to claim 9, wherein the further compound is another proteasome inhibitor, a mannosidase I inhibitor, or a compound modifying epigenome.

11. A composition for its use according to claim 10, wherein the further compound is bortezomib or MG132.

12. A composition for its use according to any of the preceding claims, wherein the composition is administered orally, intramuscularly, intraperitoneally, subcutaneously, topically, locally, or intravascularly, advantageously intravenously.


SEQUENCE LISTING

<110> INSTITUT NATIONAL DE LA SANTE ET DE LA RECHERCHE MEDICALE
      UNIVERSITE D'EVRY-VAL-D'ESSONNE
      GENETHON
      CENTRE D'ETUDE DES CELLULES SOUCHES (CECS)

<120> USE OF THIOSTREPTON OR ITS DERIVATIVES FOR THE TREATMENT OF GENETIC
DISEASES LINKED TO A PROTEIN-CONFORMATIONAL DISORDER

<130> C517-B-55401 EP

<160> 12

<170> BiSSAP 1.3.2

<210> 1
<211> 33
<212> DNA
<213> Artificial Sequence

<220>
<223> R34H primer (forward)

<400> 1
cacccacttg tgggccacgt ctttgtgcac acc                                    33


<210> 2
<211> 33
<212> DNA
<213> Artificial Sequence

<220>
<223> R34H primer (reverse)

<400> 2
ggtgtgcaca aagacgtggc ccacaagtgg gtg                                    33


<210> 3
<211> 28
<212> DNA
<213> Artificial Sequence

<220>
<223> R77C primer (forward)

<400> 3
gccccggtgg ctctgctaca cccagcgc                                          28


<210> 4
<211> 28
<212> DNA
<213> Artificial Sequence

<220>
<223> R77C primer (reverse)

<400> 4
gcgctgggtg tagcagagcc accggggc                                          28

<210> 5
<211> 27
<212> DNA
<213> Artificial Sequence

<220>
<223> I124T primer (forward)

<400> 5
ctggtgctgg agactgggga cccagaa                                          27


<210> 6
<211> 27
<212> DNA
<213> Artificial Sequence

<220>
<223> I124T primer (reverse)

<400> 6
ttctgggtcc ccagtctcca gcaccag                                          27


<210> 7
<211> 31
<212> DNA
<213> Artificial Sequence

<220>
<223> V247M primer (forward)

<400> 7
gttgactggt gcaatatgac cctggtggat a                                     31


<210> 8
<211> 31
<212> DNA
<213> Artificial Sequence

<220>
<223> V247M primer (reverse)

<400> 8
tatccaccag ggtcatattg caccagtcaa c                                     31


<210> 9
<211> 20
<212> DNA
<213> Artificial Sequence

<220>
<223> SGCA primer (forward)

<400> 9
tgctggccta tgtcatgtgc                                                  20


<210> 10
<211> 20

<212> DNA
<213> Artificial Sequence

<220>
<223> SGCA primer (reverse)

<400> 10
tctggatgtc ggaggtagcc                                                   20


<210> 11
<211> 25
<212> DNA
<213> Artificial Sequence

<220>
<223> SGCA probe

<400> 11
cgggagggaa ggctgaagag agacc                                             25


<210> 12
<211> 17
<212> PRT
<213> Artificial Sequence

<220>
<223> THSP prepeptide

<400> 12
Ile Ala Ser Ala Ser Cys Thr Thr Cys Ile Cys Thr Cys Ser Cys Ser
1               5                   10                  15
Ser